# EUROPÄISCHE PATENTSCHRIFT

(11) **EP 2 792 337 B1**
(45) Veröffentlichungstag und Bekanntmachung des Hinweises auf die Patenterteilung: **14.03.2018**
(21) Anmeldenummer: 13164211.8
(22) Anmeldetag: 17.04.2013
(51) Int. Cl.: A61F 5/01, A61F 5/37, A44B 11/25, A44B 11/28

(54) **Verschluss**
Fastener
Attache

(43) Veröffentlichungstag der Anmeldung: 22.10.2014
(73) Patentinhaber: BSN medical GmbH, 20253 Hamburg (DE)
(72) Erfinder: Grunden, Jennifer, 22769 Hamburg (DE); Schmeltzpfenning, Timo, 21244 Buchholz (DE); Bauer, Joachim, 22589 Hamburg (DE)
(74) Vertreter: Uexküll & Stolberg

(56) Entgegenhaltungen:
- DE-T5-112011 100 230
- US-A- 868 298
- US-A- 1 188 709
- US-A1- 2003 176 823
- US-A1- 2005 187 504

## Beschreibung

Die vorliegende Erfindung betrifft einen Verschluss für eine Orthese, die eine Gurtanordnung aufweist.

Orthesen zur Immobilisierung oder Fixierung eines Gelenks oder zum Stützen eines Körperteils weisen häufig eine Gurtanordnung auf, die in geeigneter Weise an den Körper des Patienten angelegt ist, um beispielsweise das fragliche Gelenk in einer vorgegebenen Stellung zu fixieren. Dabei ist es häufig erforderlich, dass über die Gurtanordnung Kräfte auf den Körper ausgeübt werden, sodass die Gurtanordnung im geschlossenen Zustand der Orthese unter einer gewissen Vorspannung stehen muss.

In diesem Zusammenhang beschreibt die DE 11 2011 100230 T5 eine Schulter- oder Armschiene, die eine Manschette und ein Gurtelement umfasst. Zur Fixierung der beiden Elemente ist ein Gurtverschluss bestehend aus zwei Teilen angebracht, wobei das erste Verschlussteil einen Zapfen und das zweite Verschlussteil eine Führungsbahn aufweist.

Die U.S. 2005/0187504 A1 zeigt eine andere Anordnung einer Orthese mit Gurtelementen zum Stützen von Körperteilen, insbesondere zum Stützen der Nacken- und Kinnpartie. Auch hier sind die Gurte über einen Verschluss bestehend aus zwei Teilen verbunden.

In der U.S. 1 188 709 A wird ebenfalls ein Verschluss offenbart, der die verschiedenen Gurte eines Bruchbandes miteinander lösbar verbindet, welches als Stütze einer Bandage am Körper dient.

Die US 868,298 A, die als nächstliegender Stand der Technik angesehen wird, beschreibt ein trennbares Verschlusssystem mit einem ersten und einem zweiten, hakenähnlichen Verschlussteil. Das zweite, hakenähnliche Verschlussteil verfügt über eine seitliche Eintrittsöffnung, die sich vom Rand des zweiten Verschlussteils schlitzartig bis zu einer zentralen Öffnung erstreckt. Hinter dem ersten Verschlussteil ist eine Schwenkplatte angeordnet, die über einen Zapfen fest mit dem ersten Verschlussteil verbunden ist. Der Zapfen erstreckt sich von dem ersten Verschlussteil weg und endet in einem Kopfabschnitt mit breiterem Durchmesser. Zum Verbinden der beiden Verschlussteile wird der Zapfen seitlich in den Schlitz des zweiten Verschlussteils eingeführt.

Auf der anderen Seite ist es aber häufig der Fall, dass der Patient bei einer schon teilweise angelegten Orthese in seiner Bewegungsfreiheit stark eingeschränkt ist und es ihm daher schwer fällt, die Gurte der Orthese in geeigneter Weise miteinander zu verbinden.

So ist es beispielsweise bei der in der DE 197 45 705 C1 beschriebenen Orthese, die dazu dient, ein Schultergelenk zu fixieren, erforderlich, dass der Patient eine Vielzahl von Gurtenden mit entsprechenden Klettverschlüssen an vorgegebenen Positionen der Gurtanordnung befestigt. Dies ist für den Patienten, der zum einen durch die angelegte Orthese in seiner Bewegungsfreiheit eingeschränkt und zum anderen zudem häufig aufgrund einer Verletzung behindert ist, mit großen Schwierigkeiten verbunden.

Darüber hinaus ergibt sich das Problem, dass die Gurtanordnung einer derartigen Orthese unter einer gewissen Vorspannung stehen muss, damit das fragliche Schultergelenk in der angelegten Stellung der Orthese zuverlässig fixiert ist.

Daher ist es ausgehend vom Stand der Technik die Aufgabe der vorliegenden Erfindung, für eine Orthese mit einer Gurtanordnung einen Verschluss bereitzustellen, der es einem Patienten, der die Orthese trägt, ermöglicht, die Gurtanordnung zum einen in einfacher Weise zu verschließen und zum anderen dabei sicherzustellen, dass mit dem Verschließen die notwendige Vorspannung innerhalb der Gurtanordnung aufgebaut wird.

Diese Aufgabe wird gemäß Anspruch 1 durch einen Verschluss gelöst mit einem ersten Verschlussteil und einem zweiten Verschlussteil, die lösbar miteinander verbunden werden können, wobei das erste und das zweite Verschlussteil Verbindungseinrichtungen für Gurte der Gurtanordnung aufweisen, wobei das erste Verschlussteil einen Zapfen aufweist, wobei das zweite Verschlussteil ein Anschlussende, an dem die Verbindungseinrichtung ausgebildet ist, und ein Kopplungsende aufweist und zwischen dem Anschlussende und dem Kopplungsende eine sich in einer Verschlussebene erstreckende Führungsbahn vorgesehen ist, die sich von einer Eintrittsöffnung zu einem Führungsbahnende erstreckt und die ausgestaltet ist, den Zapfen aufzunehmen und zwischen der Eintrittsöffnung und dem Führungsbahnende entlang der Führungsbahn zu führen, wobei die Führungsbahn derart ausgestaltet ist, dass jede mögliche Bewegung des Zapfens aus dessen Stellung im Führungsbahnende heraus entweder senkrecht zu einer Verbindungslinie zwischen dem Anschlussende und dem Kopplungsende verläuft oder die Projektion dieser Bewegung auf die Verbindungslinie zum Anschlussende weist, wobei der Zapfen einen ersten, der Verbindungseinrichtung des ersten Verschlussteils nahen Abschnitt und einen sich an den ersten Abschnitt anschließenden und an dem der Verbindungseinrichtung gegenüberliegenden Ende des ersten Abschnitts angeordneten zweiten Abschnitt aufweist, wobei die Abmessung des ersten Abschnitts senkrecht zur Erstreckungsrichtung des Zapfens der Abmessung der Führungsbahn in der Verschlussebene am Führungsbahnende entspricht, wobei die Abmessungen des zweiten Abschnitts senkrecht zur Erstreckungsrichtung des Zapfens größer ist als die Abmessung der Führungsbahn am Führungsbahnende, sodass der zweite Abschnitt eine Bewegung des Zapfens relativ zu dem zweiten Verschlussteil senkrecht zur Verschlussebene begrenzt, wenn der Zapfen am Führungsbahnende ist, wobei sich die Führungsbahn bis zum Rand des zweiten Verschlussteils erstreckt, wobei die Eintrittsöffnung im Rand des zweiten Verschlussteils ausgebildet ist, wobei das zweite Verschlussteil eine weitere Verbindungseinrichtung für einen weiteren Gurt der Gurtanordnung aufweist, wobei die Eintrittsöffnung zwischen dem Anschlussende und dem Kopplungsende auf einer ersten Seite der Verbindungslinie zwischen dem Anschlussende und dem Kopplungsende vorgesehen ist und wobei die weitere Verbindungseinrichtung auf einer zweiten, der ersten Seite gegenüberliegenden Seite der Verbindungslinie vorgesehen ist.

Ein derartig aufgebauter Verschluss lässt sich auch bei einer angelegten Orthese mit einer Gurtanordnung einfach durch den Patienten verschließen, da dieser lediglich den Zapfen im Bereich der Eintrittsöffnung in die Führungsbahn einsetzen muss und der Zapfen dann, wenn er entlang der Führungsbahn zu dem Führungsbahnende bewegt worden ist, daran gehindert ist, aus der Führungsbahn gelöst zu werden, die beiden zu verbindenden Enden der Gurtanordnung also gegeneinander verriegelt sind. Da die Abmessungen des zweiten Abschnitts des Zapfen senkrecht zu dessen Erstreckungsrichtung größer sind als die Abmessungen der Führungsbahn am Führungsbahnende, wird eine Bewegung des Zapfens senkrecht zur Ebene der Führungsbahn unabhängig von der Stellung des Zapfens relativ zum Führungsbahnende verhindert.

Die Führungsbahn ist derart ausgestaltet, dass jede mögliche Bewegung des Zapfens aus dessen Stellung im Führungsbahnende heraus entweder senkrecht zu einer Verbindungslinie zwischen dem Anschlussende und dem Kopplungsende verläuft oder die Projektion dieser Bewegung auf die Verbindungslinie zum Anschlussende weist. Diese Ausgestaltung hat zur Folge, dass bei einer Spannung, die eine Gurtanordnung einer Orthese auf den Verschluss ausübt, der Zapfen am Führungsbahnende gehalten wird.

Wenn also ein derartig aufgebauter Verschluss in einer Orthese verwendet wird, die im verschlossenen Zustand unter einer gewissen Vorspannung steht, die dann zu einer Kraft auf den Verschluss führt, die entlang der Verbindungslinie zwischen dem Anschlussende und dem Kopplungsende verläuft, ergibt sich der folgende Vorteil. Beim Verschließen der Orthese ist es lediglich erforderlich, dass der Zapfen in die entsprechend bemessene Eintrittsöffnung der Führungsbahn eingesetzt und entlang der Führungsbahn zu dem Führungsbahnende bewegt wird, wobei diese Bewegung allein durch die Form der Führungsbahn vorgegeben ist und durch die zuvor beschriebene Kraft erleichtert wird.

Denn wenn der Zapfen beabstandet von dem Führungsbahnende ist, wirkt auf das erste Verschlussteil diese Kraft, die den Zapfen zum Führungsbahnende zieht. Umgekehrt muss der Zapfen entgegen dieser Kraft vom Führungsbahnende wegbewegt werden, wenn die Orthese geöffnet werden soll. Dies gilt auch dann, wenn die Bewegung aus dem Führungsbahnende senkrecht zur Verbindungslinie verläuft, da dann zumindest Reibungskräfte wirken, die bei einer Bewegung aus dem Führungsbahnende heraus überwunden werden müssen. Somit verriegelt sich der erfindungsgemäße Verschluss bei einer Vorspannung in der Gurtanordnung selbst.

In einer weiteren bevorzugten Ausführungsform sind das Führungsbahnende und der Zapfen derart ausgestaltet, dass der erste Verschlussteil gegenüber dem zweiten Verschlussteil verschwenkbar ist, wenn der Zapfen sich am Führungsbahnende befindet. Dies ermöglicht, dass sich die beiden Teile des Verschlusses entsprechend der auf sie wirkenden Kräfte zueinander ausrichten können. Hier ist es insbesondere möglich, dass der erste Abschnitt einen kreisförmigen Querschnitt aufweist und der Durchmesser des ersten Abschnitts der Weite des Führungsbahnendes, vorzugsweise der gesamten Führungsbahn, entspricht. In diesem Zusammenhang ist unter dem Begriff der Weite die Abmessung der Führungsbahn senkrecht zu deren Verlauf bzw. senkrecht zur jeweiligen Tangente zu verstehen.

Wenn die Weite der gesamten Führungsbahn, ggf. mit Ausnahme eines Abschnitts mit einem Rastvorsprung, dem Durchmesser des Zapfens entspricht, ist dieser über die gesamte Länge der Führungsbahn geführt, kann aber verschwenkt und damit zu der wirkenden ausgerichtet werden.

Des Weiteren ist es vorteilhaft, wenn Verriegelungsmittel vorgesehen sind, um den Zapfen in der Führungsbahn, vorzugsweise am Führungsbahnende, zu halten. Insbesondere können die Verriegelungsmittel in der Weise ausgebildet sein, dass in der Führungsbahn ein Rastvorsprung ausgebildet ist, sodass die Weite der Führungsbahn im Bereich des Rastvorsprungs reduziert ist, wobei das zweite Verschlussteil derart ausgestaltet ist, dass der erste Abschnitt des Zapfens bei der Bewegung von der Eintrittsöffnung zum Führungsbahnende und umgekehrt an dem Rastvorsprung entgegen einer elastischen Gegenkraft vorbei bewegt werden muss.

Dies verhindert, dass sich der Zapfen aus der Führungsbahn heraus oder sogar vom Führungsbahnende weg hin zu der Eintrittsöffnung bewegen kann, ohne dass entweder auf den Zapfen selbst oder ein Teil des zweiten Verschlussteils eine Kraft ausgeübt werden muss. Der Zapfen ist also am Führungsbahnende in seiner Position auch mechanisch verriegelt.

Das zweite Verschlussteil weist eine weitere Verbindungseinrichtung für einen weiteren Gurt auf. Damit können an dem zweiten Verbindungsteil mehrere Gurte der Gurtanordnung der Orthese zusammenlaufen.

Ferner ist es bevorzugt, wenn benachbart zur Führungsbahn im Bereich des Führungsbahnendes, vorzugsweise benachbart zur der gesamten Führungsbahn, ein Anlageabschnitt mit einheitlicher Dicke vorgesehen ist und wobei der erste Abschnitt des Zapfens in dessen axialer Richtung eine Länge aufweist, die der Dicke des Anlageabschnitts entspricht. In diesem Fall ist das zweite Verschlussteil zwischen den Vorsprüngen am Zapfen geführt, und die Bewegung des Zapfens entlang der Führungsbahn verläuft ausschließlich in der Ebene des ebenen Abschnitts. Dies ermöglicht eine einfache Verschlussbewegung, die auch von einem in seiner Bewegung eingeschränkten Patienten leicht ausgeführt werden kann.

Ferner erstreckt sich die Führungsbahn bis zum Rand des zweiten Verschlussteils, wobei die Eintrittsöffnung im Rand des zweiten Verschlussteils ausgebildet ist. Dann kann das erste Verschlussteil mit dem zweiten in der Weise verbunden werden, dass die Verschlussbewegung ausschließlich in der Ebene des zweiten Verschlussteils erfolgt, das zweite Verschlussteil also wie ein Haken über den Zapfen gezogen wird.

Die Eintrittsöffnung ist zwischen dem Anschlussende und dem Kopplungsende auf einer ersten Seite der Verbindungslinie zwischen dem Anschlussende vorgesehen, wobei die weitere Verbindungseinrichtung auf einer zweiten, der ersten Seite gegenüberliegenden Seite der Verbindungslinie vorgesehen ist. Auf diese Weise können die beiden Verschlussteile miteinander durch eine Bewegung in der Ebene des ebenen Abschnitts in Eingriff gebracht werden, ohne dass ein an der weiteren Verbindungseinrichtung angebrachter Gurt dabei im Weg ist.

Ferner ist es bei einem solchen Aufbau bevorzugt, wenn die Projektion der Eintrittsöffnung auf der Verbindungslinie zwischen dem Anschlussende und dem Kopplungsende weiter entfernt von dem Kopplungsende ist als die Projektion des Führungsbahnendes auf die Verbindungslinie. Dadurch wird sichergestellt, dass der Zapfen aufgrund der Vorspannung in der Gurtanordnung zum Führungsbahnende und damit weg von der Eintrittsöffnung gezogen wird.

Vorzugsweise verläuft die Führungsbahn mit einer einheitlichen Krümmungsrichtung von der Eintrittsöffnung zu dem Führungsbahnende verläuft, was ein einfaches Verschließen des Verschlusses ermöglicht. Wenn dabei die Führungsbahn am Führungsbahnende derart ausgestaltet ist, dass die Bewegung des Zapfens aus der Stellung am Führungsbahnende heraus parallel zur Verbindungslinie zwischen dem Verbindungsende und dem Kopplungsende verläuft, wird sichergestellt, dass der Zapfen aufgrund der Vorspannung zuverlässig am Führungsbahnende verbleibt.

Schließlich kann in einer bevorzugten Ausführungsform der Zapfen des ersten Verschlussteils derart ausgebildet sein, dass er ein freies Ende aufweist, das mit einer in axialer Richtung des Zapfens verlaufenden Vertiefung versehen ist. Dies ermöglicht, dass der Patient den Zapfen durch Setzen eines Fingers in die Vertiefung in seiner Position festlegen oder bewegen kann, während er mit derselben Hand das zweite Verschlussteil auf den Zapfen führt. Es ist also mit einer Hand möglich, den Verschluss zu verschließen.

Des Weiteren kann der Verschluss in einer Orthese mit einer Gurtanordnung verwendet werden, wobei die Verbindungseinrichtungen mit Enden der Gurte der Gurtanordung verbunden sind.

In einer bevorzugten Ausführungsform ist die Orthese dann derart ausgestaltet, dass sie eine Zirkulargurtanordnung aufweist, die ausgestaltet ist, zirkular und horizontal um einen Patienten umzulaufen, und die einen ersten und einen zweiten Abschnitt aufweist, die vorgesehen sind, an einem Knotenpunkt lösbar verbunden zu werden, mit einer Schultergurtanordnung, die ein erstes mit der Zirkulargurtanordnung verbundenes Ende und ein zweites Ende aufweist und angepasst ist, von Posterior nach Anterior über eine Schulter des Patienten hin zu dem Knotenpunkt zu verlaufen, wobei der erste Abschnitt der Zirkulargurtanordnung mit der Verbindungseinrichtung des ersten Verschlussteils verbunden ist, wobei der zweite Abschnitt der Zirkulargurtanordnung mit der Verbindungseinrichtung des zweiten Verschlussteils verbunden ist und wobei die Schultergurtanordnung mit der weiteren Verbindungseinrichtung des zweiten Verschlussteils verbunden ist.

Bei einem derartigen Aufbau erlaubt die erfindungsgemäße Verschlusseinrichtung, durch eine einzige Schließbewegung sowohl eine Kraft in der Zirkulargurtanordnung als auch eine in der Schultergurtanordnung aufzubauen, ohne dass dies mit einer komplizierten Handhabung verbunden ist. Vielmehr kann der Patient dies auch ohne fremde Hilfe erreichen.

Die vorliegende Erfindung wird im Folgenden anhand einer ein bevorzugtes Ausführungsbeispiel zeigenden Zeichnung erläutert, wobei
- Fig. 1: das erste Verschlussteil des Ausführungsbeispiels in perspektivischer Ansicht zeigt,
- Fig. 2: das zweite Verschlussteil in Draufsicht zeigt,
- Fig. 3: das erste Verschlussteil in einer Seitenansicht zeigt,
- Fig. 4: eine perspektivische Darstellung des Ausführungsbeispiels des Verschlusses im geschlossenen Zustand zeigt,
- Fig. 5: den anterioren Teil einer Schulterorthese mit dem Ausführungsbeispiel des Verschlusses zeigt, wobei die Orthese nicht vollständig geschlossen ist,
- Fig. 6: den posterioren Teil der Orthese aus Fig. 5 zeigt,
- Fig. 7: den seitlichen Teil der Orthese aus Fig. 5 und 6 zeigt,
- Fig. 8: die Orthese aus den Fig. 5 bis 7 mit dem Ausführungsbeispiel des Verschlusses in dessen geöffneter Stellung im Detail zeigt,
- Fig. 9: die Orthese aus den Fig. 5 bis 7 mit verschlossenem Verschluss im Detail zeigt,
- Fig. 10: den anterioren Teil der Orthese aus den Fig. 5 bis 7 in vollständig verschlossenem und angelegtem Zustand zeigt,
- Fig. 11: den Ellenbogenabschnitt der Orthese aus den Fig. 5 bis 7 in einer geöffneten Stellung zeigt,
- Fig. 12: den Ellenbogenabschnitt der Orthese aus Fig. 5 bis 7 in einer geschlossenen Stellung zeigt und
- Fig. 13: den Ellenbogenabschnitt der Orthese aus den Fig. 5 bis 7 in einer seitlichen Ansicht zeigt.

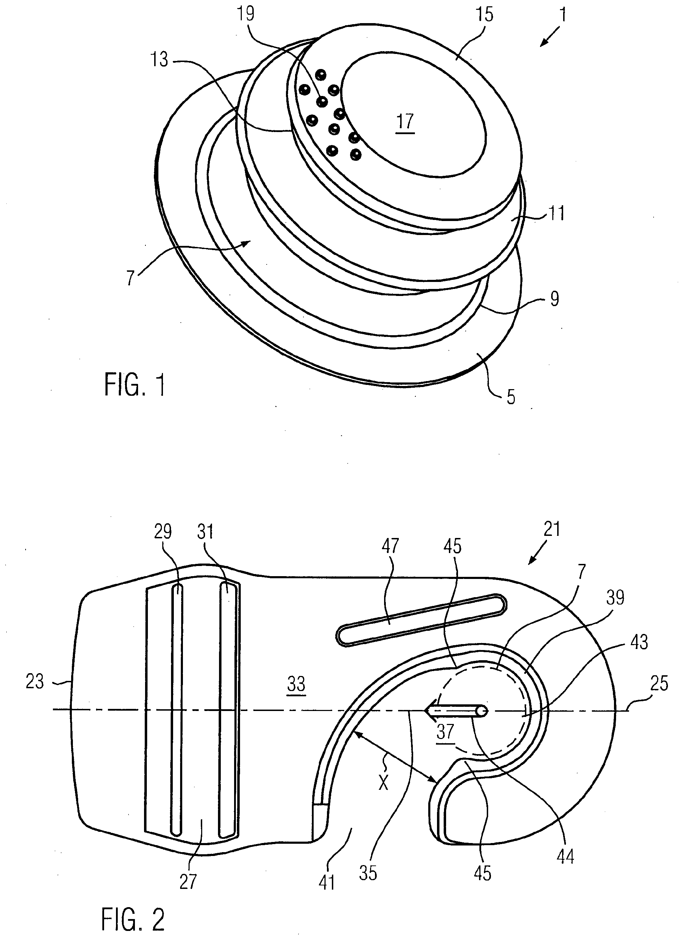

In den Fig. 1 bis 4 ist zunächst das Ausführungsbeispiel eines erfindungsgemäßen Verschlusses dargestellt, ohne dass dieser mit der Gurtanordnung einer Orthese verbunden ist.

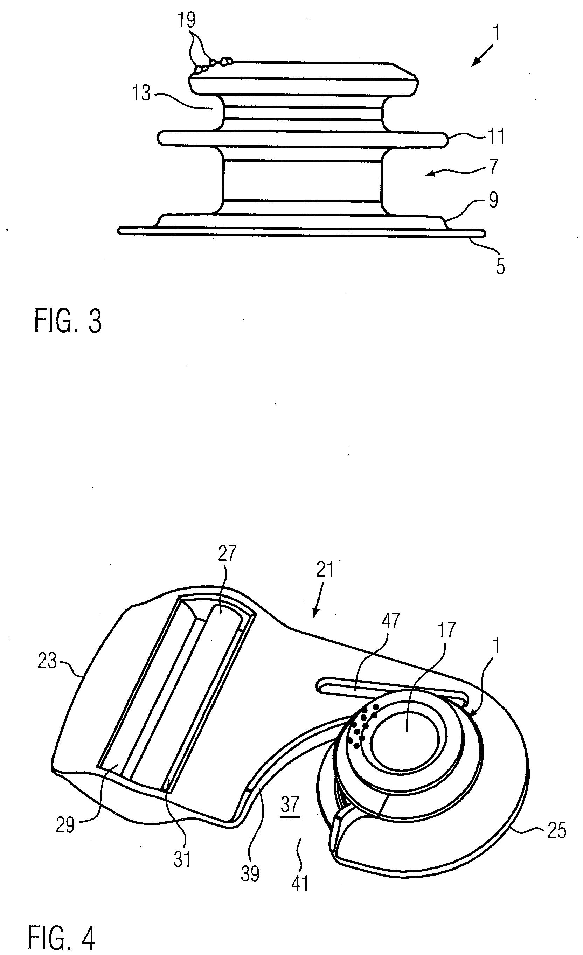

Das in den Fig. 1 und 3 dargestellte erste Verschlussteil 1 des Ausführungsbeispiels umfasst einen Zapfen 3 und weist eine als Flansch 5 ausgebildete Verbindungseinrichtung auf, über den es mit einem Ende eines Gurts einer Gurtanordnung beispielsweise durch Verschweißen, Verkleben oder Vernähen verbunden werden kann. Der Zapfen 3 des ersten Verschlussteils 1 ist mit einem ersten Abschnitt 7 versehen, der sich zwischen einem ersten Vorsprung 9 und einem als zweiten Vorsprung 11 ausgebildeten zweiten Abschnitt erstreckt. Damit ist der erste Abschnitt 7 nahe der als Flansch 5 ausgebildeten Verbindungseinrichtung angeordnet, und der zweite Abschnitt bzw. der zweite Vorsprung 11 schließt sich an dem der Verbindungseinrichtung bzw. dem Flansch 5 gegenüberliegenden Ende des ersten Abschnitts 7 an diesen an. Der Durchmesser und damit die Abmessungen der Vorsprünge 9, 11 senkrecht zur Erstreckungsrichtung des Zapfens 3 sind größer als der Durchmesser bzw. die Abmessungen des zylindrischen ersten Abschnitts 7.

Auf der von dem Flansch 5 abgewandten Seite des ersten Abschnitts 7 ist an dem Zapfen 3 ein Hinterschnitt 13 vorgesehen, der dazu dient, eine Aussparung in einem Gurtende aufzunehmen. Der Hinterschnitt 13 am freien Ende des Zapfens 3 stellt somit eine weitere Verbindungseinrichtung dar, über die beispielsweise ein weiterer Gurt lösbar mit dem ersten Verschlussteil 1 verbunden werden kann. Wie schließlich aus Fig. 1 zu erkennen ist, weist das von dem Flansch 5 abgewandte freie Ende 15 des Zapfens 3 eine sich in axialer Richtung des Zapfens 3 erstreckende Vertiefung 17 auf, die so bemessen ist, dass sie den Finger eines Patienten aufnehmen kann und das erste Verbindungsteil 1 somit in Position gehalten werden kann, wenn der Verschluss verschlossen wird. Um dabei das erste Verschlussteil 1 zusätzlich sicher halten zu können, sind an der Endfläche des freien Endes 15 um die Vertiefung 17 herum eine Vielzahl von Vorsprüngen 19 vorgesehen, die zusätzlich ein Verrutschen des Fingers des Patienten verhindern.

In Fig. 2 ist das zweite Verschlussteil 21 in Draufsicht dargestellt. Das zweite Verschlussteil 21 weist ein Anschlussende 23 sowie ein Kopplungsende 25 auf.

Im Bereich des Anschlussendes 23 ist eine erste Verbindungseinrichtung für ein Gurtende vorgesehen, die in diesem Ausführungsbeispiel derart ausgebildet ist, dass sich parallel zu einem Steg 27 ein erster Schlitz 29 und ein zweiter Schlitz 31 erstrecken, wobei der zweite Schlitz 31 weiter von dem Anschlussende 23 entfernt ist als der erste Schlitz 29. Um ein Gurtende mit der ersten Verbindungseinrichtung zu verbinden, wird dieses vom Anschlussende 23 aus entlang einer Seite des zweiten Verbindungsteils 21 und zunächst durch den zweiten Schlitz 31, dann um den Steg 27 herum und schließlich durch den ersten Schlitz 29 hindurch wieder zu dem Anschlussende 23 geführt. Dadurch, dass der erste Schlitz 29 auf der von dem Steg 27 abgewandten Seite scharfkantig begrenzt ist, kann ein Gurtende somit in sich selbst fixierender Weise mit der ersten Verbindungseinrichtung verbunden werden, wobei die Position des Gurtendes relativ zu dem zweiten Verbindungsteil 21 einstellbar ist.

Das zweite Verschlussteil 21 weist ferner einen ebenen Abschnitt 33 auf, der sich von dem Anschlussende 23 bzw. der ersten Verbindungseinrichtung 27, 29, 31 weg hin zu dem Kopplungsende 25 erstreckt. Der ebene Abschnitt 33 erstreckt sich dabei entlang einer Verbindungslinie 35 zwischen dem Anschlussende 23 und dem Kopplungsende 25. In dem ebenen Abschnitt 33 ist eine Führungsbahn 37 als Aussparung ausgebildet, die von einem Steg 39 mit konstanter Dicke ausgebildetem Anlageabschnitt umgeben ist, sodass der ebene Abschnitt 33 im Bereich um die Führungsbahn 37 mit einer einheitlichen Dicke ausgebildet ist. Durch den Steg 39 entspricht die Dicke des ebenen Abschnitts 33 im Bereich der Führungsbahn 37 dem Abstand zwischen dem ersten und zweiten Vorsprung 9, 11 bzw. der Länge des ersten Abschnitts 7 in axialer Richtung des Zapfens 3, sodass das erste Verschlussteil 1, wenn es sich in der Führungsbahn 37 befindet, sich nicht in axialer Richtung des Zapfens 3 relativ zu dem zweiten Verschlussteil 21 bewegen kann. Außerdem erstreckt sich die Führungsbahn 37 in einer durch den ebenen Abschnitt 33 definierten Verschlussebene.

Die Führungsbahn 37 hat einen einheitlich gekrümmten Verlauf und erstreckt von einer Eintrittsöffnung 41 zu einem Führungsbahnende 43, wobei die Eintrittsöffnung 41 im Rand des ebenen Abschnitts 33 und damit des zweiten Verschlussteils 21 ausgebildet ist. Die Eintrittsöffnung 41 ist unabhängig von ihrer Lage im zweiten Verschlussteil 21 derart ausgestaltet, dass durch sie das erste Verschlussteil 1 in die Führungsbahn 37 eingeführt werden kann. Da die Eintrittsöffnung 41 im Rand ausgebildet ist, kann das zweite Verschlussteil 21 einfach auf den ersten Abschnitt 9 des Zapfens 3 aufgeschoben werden.

Die Führungsbahn 37 ist damit derart ausgestaltet ist, dass jede mögliche Bewegung 44 des Zapfens 3, die Lage von dessen erstem Abschnitt 7 im Führungsbahnende 43 in Fig. 2 gestrichelt angedeutet, aus dessen Stellung im Führungsbahnende 43 so verläuft, dass zumindest die Projektion dieser Bewegung 44 auf die Verbindungslinie 35 zum Anschlussende 23 weist. In dem hier gezeigten Ausführungsbeispiel verläuft die Bewegung 44 des Zapfens 3 selbst parallel zur Verbindungslinie 35.

Es ist aber auch denkbar, dass die Bewegung geneigt zu der Verbindungslinie verläuft. In jedem Fall ist die Führungsbahn 37 am Führungsbahnende 43 aber so ausgestaltet, dass die Bewegung entweder zumindest eine Komponente hat, die zum Anschlussende 23 weist oder die Bewegung verläuft senkrecht zur Verbindungslinie 35.

Damit hat die Bewegungsbahn von dem Führungsbahnende 43 weg keine Komponente, die zum einen parallel zur Verbindungslinie 35 verläuft und zum anderen hin zu dem Kopplungsende 25 gerichtet ist. Mit anderen Worten muss sich der Zapfen 3, wenn er vom Führungsbahnende 43 hin zur Eintrittsöffnung 41 bewegt werden soll, zwangsweise weg vom Kopplungsende 25 bewegen. Um eine solche Bewegung auszuführen, muss das erste Verschlussteil 1, wenn der Verschluss in eine Gurtanordnung einer Orthese integriert ist, entgegen der Kraft bewegt werden, die durch die Vorspannung der Gurtanordnung auf den Verschluss ausgeübt wird. Dies führt dazu, dass sich der Verschluss selbst verriegelt. Es ist aber auch denkbar, dass die Bewegung des Zapfens 3 aus dem Führungsbahnende 43 heraus senkrecht zu der Verbindungslinie 35 zwischen dem Anschlussende 23 und dem Kopplungsende verläuft 25. Dann wirken, wenn der Verschluss unter Spannung steht, zumindest Reibungskräfte, die eine Bewegung des Zapfens 3 in der Führungsbahn 37 verhindern.

Wie außerdem aus Fig. 2 zu erkennen ist, verläuft die Verbindungslinie 35 im Übrigen von dem Anschlussende 23 mit der ersten Verbindungseinrichtung durch das Führungsbahnende 43 zum Kopplungsende 25, wobei die Verbindungslinie 35 auch durch die Mittelachse des Zapfens 3 bzw. des ersten Abschnitts 7 verläuft, wenn dieser sich im Führungsbahnende 43 befindet.

Die Weite X der Führungsbahn 37, und damit deren Abmessung senkrecht zur Tangente an deren Verlauf, entspricht dem Durchmesser des ersten Abschnitts 7 des ersten Verschlussteils 1 mit Ausnahme eines Bereichs zwischen Rastvorsprüngen 45, in denen die Weite reduziert ist. Allerdings ist es auch denkbar, dass die Weite der Führungsbahn 37 nicht über deren gesamte Länge den Abmessungen des ersten Abschnitts 7 entspricht, sondern beabstandet von dem Führungsbahnende 43 deutlich größer ausgebildet ist. Auf jeden Fall müssen aber die Abmessungen des zweiten Abschnitts bzw. des zweiten Vorsprungs 9 senkrecht zur Erstreckungsrichtung des Zapfens 3 größer sein als die Abmessung der Führungsbahn 37 am Führungsbahnende 43, sodass der zweite Abschnitt 9 eine Bewegung des Zapfens 3 relativ zu dem zweiten Verschlussteil 21 senkrecht zur Verschlussebene begrenzt. Unter dem Begriff des Entsprechens der Abmessungen ist in diesem Zusammenhang zu verstehen, dass der erste Abschnitt 7 und der Rand der Führungsbahn 37 im Führungsbahnende 43 nicht derart eng aneinander anliegen sollen, dass ein Verschieben des Zapfens 3 gegenüber dem zweiten Verschlussteil 21 nicht mehr möglich ist.

Die Rastvorsprünge 45 dienen dazu, das erste Verschlussteil 1 in einer Position zu verriegeln, in der es sich am Führungsbahnende 43 befindet. Dadurch muss bei diesem Ausführungsbeispiel das erste Verschlussteil 1 mit dem Zapfen 3 an den Vorsprüngen 45 vorbei bewegt werden, wobei diese entgegen einer elastischen Gegenkraft, die sich aus der Elastizität des ebenen Abschnitts 33 ergibt, aus der Führungsbahn 37 weg bewegt werden müssen.

Dadurch, dass der Zapfen 3 im Bereich des ersten Abschnitts 7 einen kreisförmigen Querschnitt hat und der Durchmesser der Weite x der Führungsbahn entspricht, kann das erste Verschlussteil 1 gegenüber dem zweiten Verschlussteil 21 verschwenkt werden.

Wie des Weiteren aus Fig. 2 zu erkennen ist, ist die Führungsbahn 37 derart ausgestaltet, dass sie den ersten Abschnitt 7 des Zapfens 3 aufnehmen und zwischen der Eintrittsöffnung 41 und dem Führungsbahnende 43 führen kann. Außerdem ist zu erkennen, dass die Eintrittsöffnung 41, die am Rand des ebenen Abschnitts 33 ausgebildet ist, derart angeordnet ist, dass deren Projektion auf die Verbindungslinie 35 weiter entfernt von dem Kopplungsende 25 ist als die Projektion des Führungsbahnendes 43 auf die Verbindungslinie 35. Somit wirkt auch an der Eintrittsöffnung 41 eine Kraft auf das erste Verschlussteil 1, wenn der Verschluss in eine Gurtanordnung integriert ist.

In dem ebenen Abschnitt 33 des zweiten Verbindungsteils ist schließlich eine zweite Verbindungseinrichtung für ein Gurtende in Form eines weiteren Schlitzes 47 ausgebildet, sodass das zweite Verschlussteil 21 mit einem zweiten Gurtende, das durch den weiteren Schlitz 47 geführt wird, verbunden werden kann. Dabei ist der weitere Schlitz 47 auf einer Seite der Verbindungslinie 35.

Wie des Weiteren aus Fig. 2 zu erkennen ist, ist die Eintrittsöffnung 41 der Führungsbahn 37 auf einer ersten Seite der Verbindungslinie 35 angeordnet, während die zweite Verbindungseinrichtung in Form des weiteren Schlitzes 47 auf einer gegenüberliegenden zweiten Seite angeordnet ist.

Wenn die Verschlussteile 1, 21 dieses Ausführungsbeispiels miteinander verbunden werden sollen und sie jeweils mit Gurtenden einer Gurtanordnung verbunden sind, muss das zweite Verbindungsteil 21 zunächst entgegen der Rückstellkräfte, die von der Gurtanordnung erzeugt werden, so weit hin zu dem ersten Verschlussteil 1 bewegt werden, dass der Zapfen 3 seitlich durch die Eintrittsöffnung 41 in die Führungsbahn 37 in dem ebenen Abschnitt 33 bewegt werden kann. Anschließend bewegt sich der Zapfen 3, in diesem Ausführungsbeispiel aufgrund der einheitlichen Krümmungsrichtung, entlang der Führungsbahn 37 und muss gegebenenfalls durch Drücken an den Vorsprüngen 45 vorbei bewegt werden, bis er das Führungsbahnende 43 erreicht. In dieser Position wird er, da dies der dem Kopplungsende 25 nächstgelegene Punkt der Führungsbahn 37 ist, aufgrund der Rückstellkräfte gehalten.

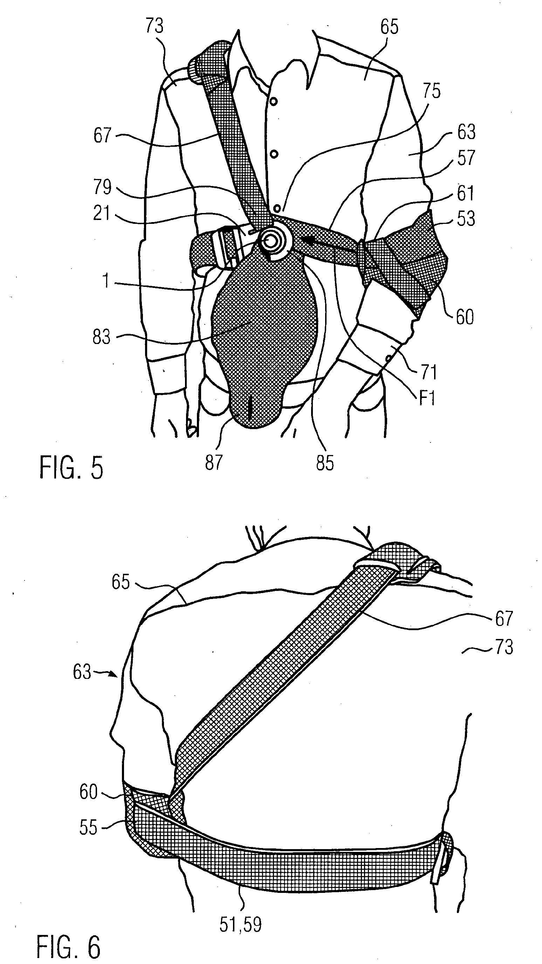

Wie aus den Fig. 5 bis 13 zu erkennen ist, ist das Ausführungsbeispiel einer Orthese, in der das zuvor beschriebene Ausführungsbeispiel eines erfindungsgemäßen Verschlusses integriert werden kann, zur Immobilisierung eines Schultergelenks vorgesehen und weist eine Gurtanordnung mit einem Zirkulargurt 51 auf, der ein erstes und ein zweites Ende 53, 55 hat (siehe Fig. 5 und 6).

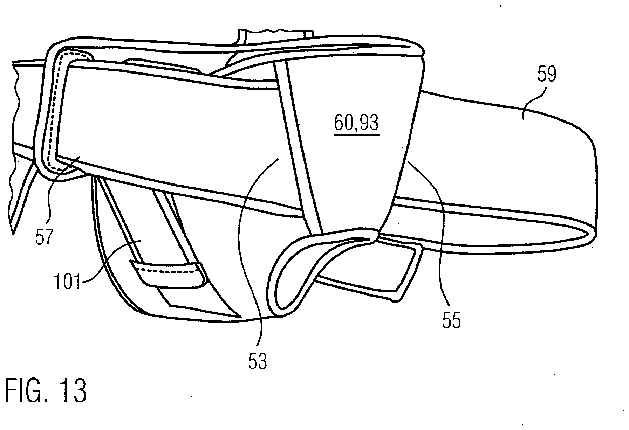

Der Zirkulargurt 51 setzt sich aus einem sich von dem ersten Ende 53 erstreckenden und anterior über den Patienten verlaufenden Anteriorabschnitt 57 und einem Posteriorabschnitt 59 zusammen, der von dem zweiten Ende 55 posterior und horizontal um den Patienten umläuft. Dabei sind bei diesem Ausführungsbeispiel das erste und das zweite Ende 53, 55 des Zirkulargurts 51 in der Weise miteinander verbunden, dass sie an einer Aufnahme 60 für den Ellenbogen benachbart zueinander befestigt sind, wie dies insbesondere in Fig. 13 gezeigt ist. Bei diesem Ausführungsbeispiel bildet das erste Ende 53 des Zirkulargurts 51 mit der Aufnahme 60 einen ersten Gurtabschnitt, der einen Armabschnitt 61 des Arms 63 des Patienten teilweise umschlingt, der sich von dem ruhigzustellenden ersten Schultergelenk 65 erstreckt.

Dabei wird unter dem Begriff "Armabschnitt" im Zusammenhang mit der vorliegenden Erfindung der Bereich des von dem verletzten ersten Schultergelenk 65 ausgehenden Arms 63 verstanden, der den Ellenbogen selbst sowie die dazu benachbarten Bereiche umfasst.

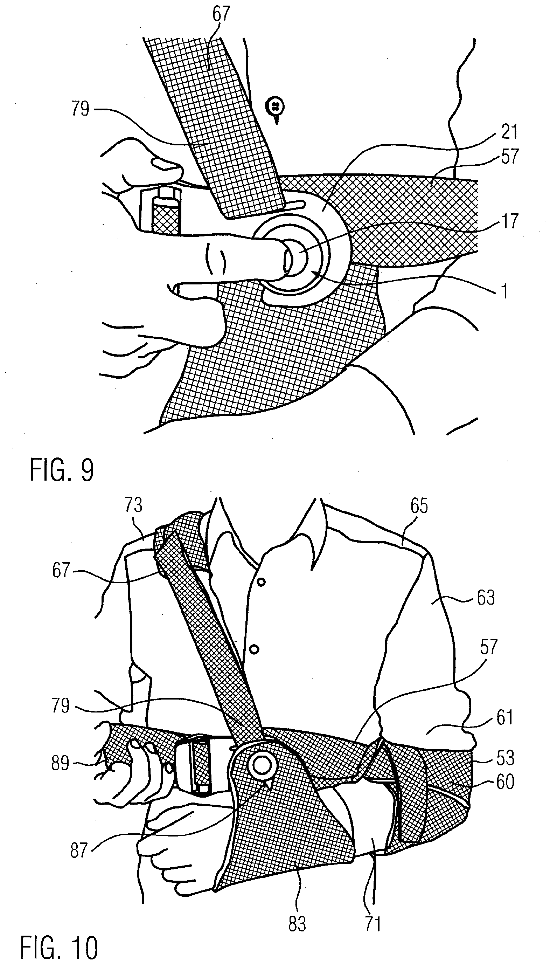

Aus den Fig. 5, 6 und 7 geht zudem hervor, dass bei dieser Orthese ein Schultergurt 67 vorgesehen ist, dessen erstes Ende 69 einen zweiten Gurtabschnitt bildet, wobei das erste Ende 69 mit der Aufnahme 60 verbunden ist und so den Armabschnitt 61 des Arms 63 des Patienten ebenfalls darüber umschlingt. Dabei geht das erste Ende 69 am Unterarm 71 benachbart zu dem Ellenbogen in die Aufnahme 60 über. Der Schultergurt 69 erstreckt sich posterior und diagonal über den Rücken des Patienten zu der dem ersten Schultergelenk 65 contralateralen Schulter 73. Wie die Fig. 5 und 10 zeigen, verläuft der Schultergurt 67 von der contralateralen Schulter 73 in vertikaler Richtung anterior zu dem Zirkulargurt 51 und ist dort in einem ersten Verbindungspunkt mit diesem über die Verschlussteile 1, 21 verbunden.

Wie den Fig. 5 bis 13 außerdem zu entnehmen ist, ist der Verschluss mit den Verschlussteilen 1, 21 in geschlossenem Zustand der Orthese im Bereich eines virtuellen Knotenpunkts 75 angeordnet (siehe Fig. 5).

Dabei ist das zweite Verschlussteil 21 mit dem zweiten Ende 79 des Schultergurts 67 in der Weise verbunden, dass das zweite Ende 79 durch den weiteren Schlitz 47 gezogen und das zweite Verschlussteil 21 damit entlang des Schultergurts 67 verschoben werden kann. Zum Verschieben des zweiten Endes 79 des Schultergurts 67 muss zunächst eine Klettverbindung geöffnet werden, und dann kann das zweite Ende 79 mehr oder weniger weit durch den weiteren Schlitz 47 gezogen werden.

Außerdem ist das zweite Verschlussteil 21 mit dem Posteriorabschnitt 59 des Zirkulargurts 51 verbunden, wobei das zweite Verschlussteil 21 ebenfalls in dessen Längsrichtung verschiebbar ist. Hierzu ist der Posteriorabschnitt 59 in der schon beschriebenen Weise durch den ersten und zweiten Schlitz 29, 31 sowie um den Steg 27 geführt.

Mittels des zweiten Verschlussteils 21 sind somit das zweite Ende 79 des Schultergurts 67 und der Zirkulargurt 51 bzw. dessen Posteriorabschnitt 59 miteinander verbunden. Ferner ist dadurch, dass das zweite Verschlussteil 21 verschiebbar an dem Zirkulargurt 51 und dem Schultergurt 67 angebracht ist, ein erster Verbindungspunkt, an dem der Schultergurt 67 und der Zirkulargurt 51 miteinander verbunden sind, entlang des Zirkulargurts 51 und entlang des Schultergurts 67 verschiebbar.

Das erste Verschlussteil 1 ist an dem freien Ende 81 des Anteriorabschnitts 57 in der Weise befestigt, dass der Flansch 5 mit dem freien Ende 81 vernäht ist. Es ist aber auch denkbar, dass der Flansch 5 mit dem freien Ende 81 verklebt oder verschweißt ist.

Der erste Abschnitt 7 des Zapfens 3 kann in die Eintrittsöffnung 41 eingeschoben und entlang der Führungsbahn 37 bis zum Führungsbahnende 43 bewegt werden, um den aus den Verschlussteilen 1, 21 gebildeten Verschluss zu schließen. Dabei ist zu erkennen, dass sowohl in der geschlossenen als auch in der geöffneten Stellung der Schultergurt 67 und der Posteriorabschnitt 59 miteinander verbunden sind.

Fig. 5 und 13 zeigen ferner, dass ein Haltegurt 83 vorgesehen ist, dessen erstes Ende 85 mit dem freien Ende 81 des Anteriorabschnitts 57 verbunden ist. Das zweite Ende 87 des Haltegurts 83 kann lösbar mit dem ersten Verbindungsteil 1 verbunden werden, indem eine Aussparung 88 in dem zweiten Ende 87 in den Bereich des Hinterschnitts 13 gedrückt und so an dem freien Ende 15 des Zapfens 3 befestigt wird, sodass der Haltegurt 83 eine Schlaufe bildet, in die das Handgelenk des Unterarms 71, der von der dem ersten Schultergelenk 65 ausgeht, aufgenommen werden kann (siehe Fig. 10). Wie weiter aus Fig. 13 zu erkennen ist, verlaufen an dem Haltegurt 83 senkrecht zu dessen Erstreckungsrichtung Verstärkungsstreben 91, die ein Abknicken des Haltegurts 83 verhindern, sodass ein vom Haltegurt 83 aufgenommenes Handgelenk nicht abknicken kann.

Durch die am Anschlussende 23 des zweiten Verschlussteils 21 vorgesehenen zwei Schlitze 29, 31 und den dazwischen angeordneten Steg 27 ist eine Fixiervorrichtung vorgesehen, um die Lage des ersten Verbindungspunkts, an dem der Schultergurt 67 und der Posteriorabschnitt 59 miteinander verbunden sind, entlang des Posteriorabschnitts 59 festzulegen. Diese Fixiervorrichtung ist bei dem zuvor beschriebenen Aufbau derart ausgestaltet, dass der erste Verbindungspunkt durch Ziehen an dem vom zweiten Ende 55 des Zirkulargurts 51 bzw. des Posteriorabschnitts 59 entfernten Posteriorabschnittende 89 hin zu dem zweiten Ende 55 verschoben werden kann, während eine Bewegung des ersten Verbindungspunkts hin zum Posteriorabschnittende 89 durch Ziehen an dem zweiten Ende 55 blockiert wird. Dies bedeutet, dass der Posteriorabschnitt 59 durch Ziehen an dessen Ende 89 durch die Schlitze 29, 31 hindurch durch das Anschlussende 23 gezogen werden kann, während eine Bewegung des Posteriorabschnitts 59 in entgegengesetzter Richtung blockiert ist.

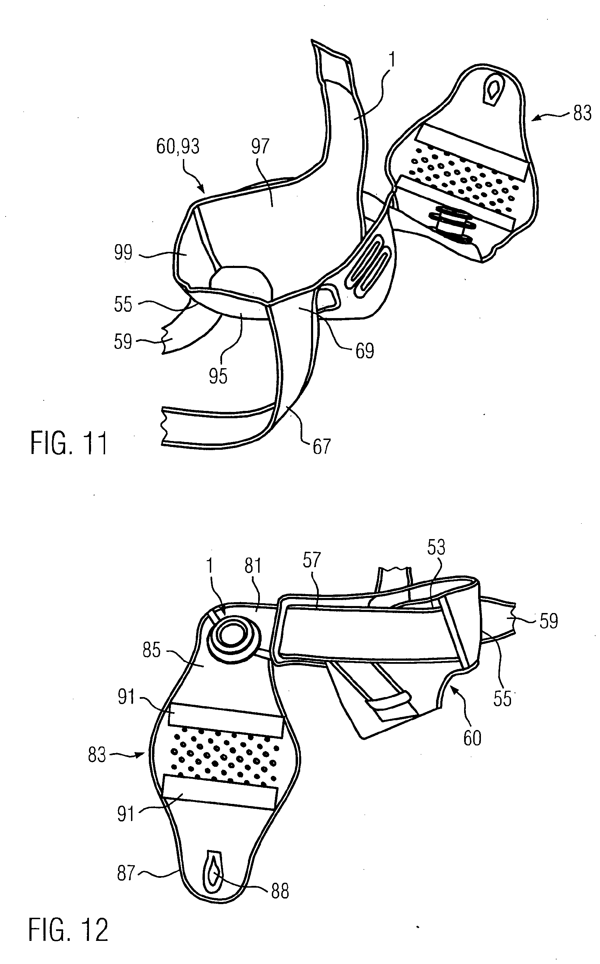

Wie schließlich den Fig. 11 bis 13 zu entnehmen ist, weist die Aufnahme 60 für den Ellenbogen eine Einrichtung auf, um deren Weite einstellen zu können. Die Aufnahme des Ellenbogens hat einen Hauptkörper 93, der eine am Körper des Patienten anliegende Innenfläche 95, eine vom Körper weg angeordneten Außenfläche 97 und eine außen am Oberarm anliegende Seitenfläche 99 umfasst. Dabei sind die Innenfläche 95 und die Außenfläche 97 an einer Kante, die am Unterarm anliegt, miteinander verbunden. An der Außenfläche 97 ist an der entfernt zu der Verbindung mit der Innenfläche 95 gelegenen Kante ein Verstellgurt 101 befestigt, der durch zwei Öffnungen 103 in der Innenfläche und über den Unterarm geführt und wiederum lösbar an der Außenfläche 97 befestigt werden kann. Durch den Verstellgurt 101 ist es möglich, die Weite der Aufnahme 60 an die Bedürfnisse des Patienten anzupassen.

Das zuvor beschriebene Ausführungsbeispiel einer Orthese wird nun wie folgt angelegt.

Zuerst wird der Armabschnitt 61 in der mit den Enden 53, 69 des Zirkulargurts 51 und des Schultergurts 67 verbundenen Aufnahme 60 aufgenommen, sodass diese dann den Armabschnitt 61 über die Aufnahme 60 umschlingen. Dabei ist der Verstellgurt 101 über den Unterarm des Patienten geführt.

Da das zweite Ende 79 des Schultergurts 67 und der Posteriorabschnitt 59 über das zweite Verschlussteil 21 auch in der geöffneten Stellung des Verschlusses miteinander verbunden sind, bilden der Posteriorabschnitt 59 und der Schultergurt 67 zunächst eine Schlaufe, in die die zum ersten Schultergelenk 65 contralaterale Schulter 73 aufgenommen werden kann bzw. der Patient muss lediglich diese Schlaufe über die contralaterale Schulter 73 hängen.

Anschließend wird der aus den Verschlussteilen 1, 21 gebildetet Verschluss in der Weise geschlossen, dass der Zapfen 3 in die Eintrittsöffnung 41 eingesetzt wird und sich dieser dann aufgrund der am freien Ende 81 des Anteriorabschnitts 57 wirkenden Kräfte zum Führungsbahnende 43 gezogen wird, wobei hierbei allerdings durch die Rastvorsprünge 45 entgegen der auf sie wirkenden Gegenkräfte aus der Führungsbahn 37 gedrückt werden müssen. Dabei zeigt insbesondere Fig. 9, dass die Vertiefung 17 im freien Ende 15 des Zapfens 3 ein Verschließen des Verschlusses mit einer Bewegung des Zapfens 3 entlang der Führungsbahn 37 mit nur einer Hand ermöglicht.

Wenn sich der Zapfen 3 am Führungsbahnende 43 befindet, wird dieser aufgrund des beschriebenen Verlaufs der Führungsbahn 37 und der entlang der Verbindungslinie wirkenden Kraft, die sich aus der Vorspannung in dem Zirkulargurt 51 und dem Schultergurt 67 ergibt, am Führungsbahnende 43 gehalten.

Schließlich kann dann der Zirkulargurt 51 zusätzlich durch Ziehen an dem Posteriorabschnittende 89 gespannt werden, wobei das zweite Verschlussteil 21 in der zuvor beschriebenen Weise ausgestaltet ist, sodass eine Rückbewegung des Posteriorabschnitts 59 nicht möglich ist. Danach ergibt sich die in Fig. 5 dargestellte Situation.

Schließlich kann der Patient den Unterarm 71 in der Weise fixieren, dass der Haltegurt 83 um den Unterarm geschlungen wird, wobei das zweite Ende 87 des Haltegurts 83 am Zapfen 3 des ersten Verschlussteils 1 befestigt wird (siehe Fig. 10).

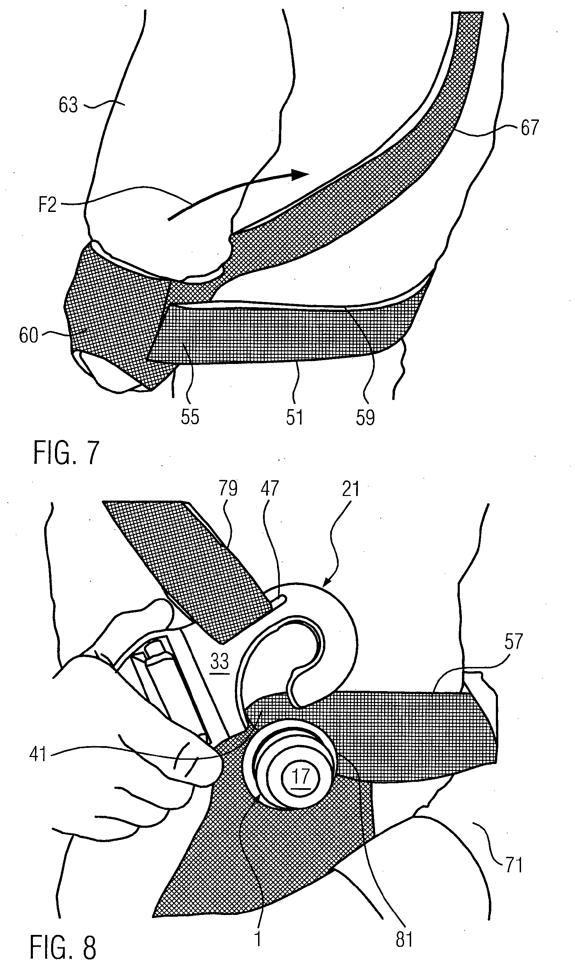

Bei zuvor beschriebenen Orthese sind demnach ein erster und ein zweiter Gurtabschnitt, nämlich das erste Ende 53 des Zirkulargurts 51 und das erste Ende 69 des Schultergurts 67, vorgesehen, die an dem Armabschnitt 61 in der Weise angreifen, dass sie ihn zumindest teilweise mit Hilfe der Aufnahme 60 umschlingen, sodass durch Zug in der Erstreckungsrichtung der Gurtabschnitte der Armabschnitt 61 mit einer ersten und einer zweiten Kraft beaufschlagt wird. Der erste Gurtabschnitt bzw. das erste Ende 53 des Zirkulargurts 51 übt im angelegten Zustand der Orthese, in der der von dem verletzten Schultergelenk 65 ausgehende Arm 63 seitlich bzw. frontal am Körper anliegt, eine Kraft (F1; siehe Fig. 5) auf den Armabschnitt 61 aus, die zu dem Körper hin gerichtet ist und die vorzugsweise im Wesentlichen parallel zu der Frontalebene des Patienten verläuft, aber wenigstens eine erste Komponente hat, die horizontal und parallel zur Frontalebene verläuft. Dadurch wird aufgrund des ersten Gurtabschnitts der Armabschnitt 61 an den Körper herangezogen, sodass die erste Kraftkomponente adduzierend auf den ersten Arm 63 wirkt. Gleichzeitig übt der zweite Gurtabschnitt, d.h. das erste Ende 69 des Schultergurts 67 eine zweite Kraft (F2; siehe Fig. 7) auf den Armabschnitt 61 aus, die wenigstens eine Komponente hat, die horizontal und parallel zur Sagittalebene und damit senkrecht zu der ersten Komponente verläuft und nach posterior gerichtet ist, sodass eine leichte Retroversion des Arms 63 bewirkt wird. Aufgrund dieser Kombination der zwei Kraftkomponenten wird der Arm 63 zuverlässig am Körper fixiert.

Außerdem ergibt sich der Vorteil, dass das erste, zu immobilisierende Schultergelenk 65 sowohl bei gestrecktem als auch bei gebeugtem Ellenbogen immobilisiert ist.

Des Weiteren zeigt sich, dass der erfindungsgemäße Verschluss aus den beiden eingangs beschriebenen Verschlussteilen 1, 21 für den Patienten mit dem großen Vorteil verbunden, dass allein durch deren Lösen, also das Überführen von der geschlossenen in die geöffnete Stellung, die Wirkung beider Kräfte F1, F2 auf den Armabschnitt 61 aufgehoben wird, sodass der von dem verletzten Schultergelenk 65 ausgehende Arm 63 sofort freigegeben ist. Umgekehrt kann allein durch das Schließen des Verschlusses 1, 21 sofort die Wirkung der beiden Kräfte F1, F2 erreicht werden, ohne dass weitere Maßnahmen durch den Patienten erforderlich sind. Dies vereinfacht das Anlegen erheblich.

Allgemein umfasst die zuvor beschriebene Orthese somit eine Zirkulargurtanordnung 51, 57, 59, die ausgestaltet ist, zirkular und horizontal um einen Patienten umzulaufen, und die einen ersten und einen zweiten Abschnitt in Form des Posterior- und des Anteriorabschnitts 57, 59 aufweist, die vorgesehen sind, an einem Knotenpunkt 75 lösbar verbunden zu werden. Ferner weist diese Orthese eine Schultergurtanordnung 67 auf, die ein erstes mit der Zirkulargurtanordnung 51, 57, 59 verbundenes Ende 69 und ein zweites Ende aufweist und die angepasst ist, von Posterior nach Anterior über eine Schulter des Patienten hin zu dem Knotenpunkt 75 zu verlaufen. Am Knotenpunkt 75 ist der erste Abschnitt der Zirkulargurtanordnung 51, 57, 59, nämlich der Anteriorabschnitt 57, mit der Verbindungseinrichtung des ersten Verschlussteils 1 verbunden ist, während der zweite Abschnitt, nämlich der Posteriorabschnitt 59 mit der Verbindungseinrichtung 27, 29, 31 des zweiten Verschlussteils 21 verbunden ist. Schließlich ist die Schultergurtanordnung 67 mit der weiteren Verbindungseinrichtung 47 des zweiten Verschlussteils 21 verbunden ist.

Dabei ist durch den Aufbau der Verschlussteile 1, 21 gegeben, dass der Verschluss in einfacher Weise, ggf. nur mit einer Hand, verschlossen werden kann und er sich allein schon aufgrund des Verlaufs der Führungsbahn 37 in dieser Stellung hält. Damit kann eine mit dem erfindungsgemäßen Verschluss versehene Orthese auch von einem in seiner Bewegungsfreiheit eingeschränkten Patienten angelegt und verschlossen werden. Dies gilt nicht nur für die zuvor beschriebene Orthese zum Fixieren einer Schulter, sondern der Verschluss kann insbesondere auch bei anderen Orthesen, die eine Zirkulargurt- und eine Schultergurtanordnung aufweisen, wie beispielsweise Armabduktionskissen oder dgl., gewinnbringend eingesetzt werden.

## Patentansprüche

1. Verschluss für eine Orthese, die eine Gurtanordnung aufweist,
mit einem ersten Verschlussteil (1) und einem zweiten Verschlussteil (21), die lösbar miteinander verbunden werden können, wobei das erste und das zweite Verschlussteil (1, 21) Verbindungseinrichtungen (5, 27, 29, 31, 47) für Gurte der Gurtanordnung aufweisen,
wobei das erste Verschlussteil (1) einen Zapfen (3) aufweist,
wobei das zweite Verschlussteil (21) ein Anschlussende (23), an dem die Verbindungseinrichtung (27, 29, 31) ausgebildet ist, und ein Kopplungsende (25) aufweist und zwischen dem Anschlussende (23) und dem Kopplungsende (25) eine sich in einer Verschlussebene erstreckende Führungsbahn (37) vorgesehen ist, die sich von einer Eintrittsöffnung (41) zu einem Führungsbahnende (43) erstreckt und die ausgestaltet ist, den Zapfen (3) aufzunehmen und zwischen der Eintrittsöffnung (41) und dem Führungsbahnende (43) entlang der Führungsbahn (37) zu führen,
wobei die Führungsbahn (37) derart ausgestaltet ist, dass jede mögliche Bewegung (44) des Zapfens (3) aus dessen Stellung im Führungsbahnende (43) heraus entweder senkrecht zu einer Verbindungslinie (35) zwischen dem Anschlussende (23) und dem Kopplungsende verläuft (25) oder die Projektion dieser Bewegung (44) auf die Verbindungslinie (35) zum Anschlussende (23) weist,
wobei der Zapfen (3) einen ersten, der Verbindungseinrichtung (5) des ersten Verschlussteils (1) nahen Abschnitt (7) und einen sich an den ersten Abschnitt (7) anschließenden und an dem der Verbindungseinrichtung (5) gegenüberliegenden Ende des ersten Abschnitts (7) angeordneten zweiten Abschnitt (11) aufweist,
wobei die Abmessung des ersten Abschnitts (7) senkrecht zur Erstreckungsrichtung des Zapfens (3) der Abmessung der Führungsbahn (37) in der Verschlussebene am Führungsbahnende (43) entspricht,
wobei die Abmessungen des zweiten Abschnitts (11) senkrecht zur Erstreckungsrichtung des Zapfens (3) größer sind als die Abmessung der Führungsbahn (37) am Führungsbahnende (43), sodass der zweite Abschnitt (11) eine Bewegung des Zapfens (3) relativ zu dem zweiten Verschlussteil (21) senkrecht zur Verschlussebene begrenzt,
wobei sich die Führungsbahn (37) bis zum Rand des zweiten Verschlussteils (21) erstreckt, wobei die Eintrittsöffnung (41) im Rand des zweiten Verschlussteils (21) ausgebildet ist, und wobei die Eintrittsöffnung (41) zwischen dem Anschlussende (23) und dem Kopplungsende (25) auf einer ersten Seite der Verbindungslinie (35) zwischen dem Anschlussende (23) und dem Kopplungsende (25) vorgesehen ist,
**dadurch gekennzeichnet, dass** das zweite Verschlussteil (21) eine weitere Verbindungseinrichtung (47) für einen weiteren Gurt der Gurtanordnung aufweist, und wobei die weitere Verbindungseinrichtung (47) auf einer zweiten, der ersten Seite gegenüberliegenden Seite der Verbindungslinie (35) vorgesehen ist.

2. Verschluss nach Anspruch 1, wobei das Führungsbahnende (43) und der Zapfen (3) derart ausgestaltet sind, dass das erste Verschlussteil (1) gegenüber dem zweiten Verschlussteil (21) verschwenkbar ist, wenn der Zapfen (3) sich am Führungsbahnende (43) befindet.

3. Verschluss nach Anspruch 1 oder 2, wobei Verriegelungsmittel (45) vorgesehen sind, um den Zapfen in der Führungsbahn (37), vorzugsweise am Führungsbahnende (43), zu halten.

4. Verschluss nach Anspruch 3, wobei in der Führungsbahn (37) ein Rastvorsprung (45) ausgebildet ist, sodass die Weite der Führungsbahn (37) im Bereich des Rastvorsprungs (45) reduziert ist, und
wobei das zweite Verschlussteil (21) derart ausgestaltet ist, dass der erste Abschnitt des Zapfens (3) bei der Bewegung von der Eintrittsöffnung (41) zum Führungsbahnende (43) und umgekehrt an dem Rastvorsprung (45) entgegen einer elastischen Gegenkraft vorbei bewegt werden muss.

5. Verschluss nach einem der Ansprüche 1 bis 4, wobei benachbart zur Führungsbahn (37) im Bereich des Führungsbahnendes (43), vorzugsweise benachbart zu der gesamten Führungsbahn (37), ein Anlageabschnitt (39) mit einheitlicher Dicke vorgesehen ist und
wobei der erste Abschnitt (7) des Zapfens (3) in dessen axialer Richtung eine Länge aufweist, die der Dicke des Anlageabschnitts (39) entspricht.

6. Verschluss nach Anspruch 1, wobei die Projektion der Eintrittsöffnung (41) auf die Verbindungslinie (35) zwischen dem Anschlussende (23) und dem Kopplungsende (25) weiter entfernt von dem Kopplungsende (25) ist als die Projektion des Führungsbahnendes (43) auf die Verbindungslinie (35) .

7. Verschluss nach Anspruch 6, wobei die Führungsbahn (37) mit einer einheitlichen Krümmungsrichtung von der Eintrittsöffnung (41) zu dem Führungsbahnende (43) verläuft.

8. Verschluss nach Anspruch 7, wobei die Führungsbahn (37) am Führungsbahnende (43) derart ausgestaltet ist, dass die Bewegung (44) des Zapfens (3) aus der Stellung am Führungsbahnende (43) heraus parallel zur Verbindungslinie (35) zwischen dem Verbindungsende (23) und dem Kopplungsende (25) verläuft.

9. Verschluss nach einem der Ansprüche 1 bis 8, wobei das erste Verschlussteil (1) derart ausgebildet ist, dass sich der Zapfen (3) von der Verbindungseinrichtung weg erstreckt und ein freies Ende (15) aufweist und
wobei das freie Ende eine sich in axialer Richtung des Zapfens (5) erstreckende Vertiefung (17) aufweist.

10. Orthese mit einer Gurtanordnung und mit einem Verschluss nach einem der Ansprüche 1 bis 9, wobei die Verbindungseinrichtungen mit den Gurten der Gurtanordung verbunden sind.

11. Orthese nach Anspruch 10 mit einer Zirkulargurtanordnung (51, 57, 59), die ausgestaltet ist, zirkular und horizontal um einen Patienten umzulaufen, und die einen ersten und einen zweiten Abschnitt (57, 59) aufweist, die vorgesehen sind, an einem Knotenpunkt (75) lösbar verbunden zu werden,
mit einer Schultergurtanordnung (67), die ein erstes mit der Zirkulargurtanordnung (51, 57, 59) verbundenes Ende (69) und ein zweites Ende aufweist und angepasst ist, von Posterior nach Anterior über eine Schulter des Patienten hin zu dem Knotenpunkt (75) zu verlaufen,
wobei der erste Abschnitt (57) der Zirkulargurtanordnung (51, 57, 59) mit der Verbindungseinrichtung des ersten Verschlussteils (1) verbunden ist,
wobei der zweite Abschnitt (59) der Zirkulargurtanordnung (51, 57, 59) mit der Verbindungseinrichtung (27, 29, 31) des zweiten Verschlussteils (21) verbunden ist und
wobei die Schultergurtanordnung (67) mit der weiteren Verbindungseinrichtung (47) des zweiten Verschlussteils (21) verbunden ist.

## Claims

1. A closure for an orthosis, having a belt assembly,
having a first closure part (1) and a second closure part (21), which can be releasably connected to one another, wherein the first and the second closure parts (1, 21) have connecting means (5, 27, 29, 31, 47) for belts of the belt assembly,
wherein the first closure part (1) has a pin (3), wherein the second closure part (21) has a connection end (23), on which the connecting means (27, 29, 31) is formed, and has a coupling end (25), and between the connection end (23) and the coupling end (25) a guide track (37) extending in a closure plane is provided, which extends from an entrance opening (41) to a guide track end (43), and which is designed to receive the pin (3) and guide it between the entrance opening (41) and the guide track end (43) along the guide track (37),
wherein the guide track (37) is designed such that any possible movement (44) of the pin (3) out of its position in the guide track end (43) either runs perpendicular to a connecting line (35) between the connection end (23) and the coupling end (25), or the projection of this movement (44) is directed on the connecting line (35) toward the connection end (23),
wherein the pin (3) has a first section (7) near to the connecting means (5) of the first closure part (1) and a second section (11) adjoining the first section (7) and disposed on the end of the first section (7) opposite the connecting means (5),
wherein the dimension of the first section (7) perpendicular to the direction of extension of the pin (3) corresponds to the dimension of the guide track (37) in the closure plane at the guide track end (43),
wherein the dimensions of the second section (11) perpendicular to the direction of extension of the pin (3) are greater than the dimension of the guide track (37) at the guide track end (43), so that the second section (11) restricts a movement of the pin (3) relative to the second closure part (21) perpendicular to the closure plane,
wherein the guide track (37) extends to the edge of the second closure part (21), wherein the entrance opening (41) is formed in the edge of the second closure part (21), and
wherein the entrance opening (41) is provided between the connection end (23) and the coupling end (25) on a first side of the connecting line (35) between the connection end (23) and the coupling end (25),
**characterized in that** the second closure part (21) has another connecting means (47) for another belt of the belt assembly,
and wherein the other connecting means (47) is provided on a second side of the connecting line (35) opposite the first side.

2. The closure according to Claim 1, wherein the guide track end (43) and the pin (3) are designed such that the first closure part (1) is pivotable relative to the second closure part (21), when the pin (3) is located on the guide track end (43).

3. The closure according to one of Claims 1 or 2, wherein locking means (45) are provided in order to hold the pin in the guide track (37), preferably at the guide track end (43).

4. The closure according to Claim 3, wherein a latching projection (45) is formed in the guide track (37), so that the width of the guide track (37) is reduced in the region of the latching projection (45), and
wherein the second closure part (21) is designed such that the first section of the pin (3), when moving from the entrance opening (41) to the guide track end (43) and back again, must be moved past the latching projection (45), against an elastic counterforce.

5. The closure according to one of Claims 1 to 4, wherein an abutment section (39) with uniform thickness is provided adjacent to the guide track (37) in the region of the guide track end (43), preferably adjacent to the entire guide track (37), and
wherein the first section (7) of the pin (3) has, in its axial direction, a length corresponding to the thickness of the abutment section (39).

6. The closure according to Claim 1, wherein the projection of the entrance opening (41) onto the connecting line (35) between the connection end (23) and the coupling end (25) is further spaced from the coupling end (25) than the projection of the guide track end (43) onto the connecting line (35).

7. The closure according to Claim 6, wherein the guide track (37) extends with a uniform direction of curvature from the entrance opening (41) to the guide track end (43).

8. The closure according to Claim 7, wherein the guide track (37) is designed at the guide track end (43) such that the movement (44) of the pin (3) out of the position on the guide track end (43) runs parallel to the connecting line (35) between the connection end (23) and the coupling end (25).

9. The closure according to one of Claims 1 to 8, wherein the first closure part (1) is designed such that the pin (3) extends away from the connecting means and has a free end (15) and
wherein the free end has an indentation (17) extending in the axial direction of the pin (5).

10. An orthosis having a belt assembly and having a closure according to one of Claims 1 to 9, wherein the connecting means are connected to the belts of the belt assembly.

11. The orthosis according to Claim 10, having a circular belt assembly (51, 57, 59), which is designed to pass around a patient in a circular and horizontal manner, and which has a first and a second section (57, 59), which are designed to be detachably connected at a point of intersection (75),
having a shoulder belt assembly (67), which has a first end (69) connected to the circular belt assembly (51, 57, 59) and a second end, and which is adapted so as to run from the back to the front over a shoulder of the patient to the point of intersection (75),
wherein the first section (57) of the circular belt assembly (51, 57, 59) is connected to the connecting means of the first closure part (1),
wherein the second section (59) of the circular belt assembly (51, 57, 59) is connected to the connecting means (27, 29, 31) of the second closure part (21), and
wherein the shoulder belt assembly (67) is connected to the other connecting means (47) of the second closure part (21).

## Revendications

1. Fermeture pour une orthèse, qui comprend un dispositif à sangles,
avec une première partie de fermeture (1) et une deuxième partie de fermeture (21), qui peuvent être reliées entre elles de manière amovible, la première et la deuxième partie de fermeture (1, 21) comprenant des dispositifs de liaison (5, 27, 29, 31, 47) pour les sangles du dispositif à sangles,
la première partie de fermeture (1) comprenant un pivot (3),
la deuxième partie de fermeture (21) comprenant une extrémité de raccordement (23) à laquelle le dispositif de liaison (27, 29, 31) est disposé, et une extrémité de couplage (25) et, entre l'extrémité de raccordement (23) et l'extrémité de couplage (25), une piste de guidage (37), s'étendant dans un plan de fermeture, étant prévue, qui s'étend d'une ouverture d'entrée (41) vers une extrémité de piste de guidage (43) et qui est conçue pour loger le pivot (3) et le guider entre l'ouverture d'entrée (41) et l'extrémité de piste de guidage (43) le long de la piste de guidage (37),
la piste de guidage (37) étant conçue de façon à ce que chaque mouvement possible (44) du pivot (3) hors de sa position dans l'extrémité de piste de guidage (43) s'effectue soit perpendiculairement à une ligne de liaison (35) entre l'extrémité de raccordement (23) et l'extrémité de couplage (25) soit la projection de ce mouvement (44) est orientée sur la ligne de liaison (35) vers l'extrémité de raccordement (23),
le pivot (3) comprenant une première portion (7), proche du dispositif de liaison (5) de la première partie de fermeture (1) et une deuxième portion (11), prolongeant la première portion (7) et disposée sur l'extrémité de la première portion (7) opposée au dispositif de liaison (5),
les dimensions de la première portion (7) perpendiculairement à la direction d'extension du pivot (3) correspondant aux dimensions de la piste de guidage (37) dans le plan de fermeture au niveau de l'extrémité de la piste de guidage (43),
les dimensions de la deuxième portion (11) perpendiculairement à la direction d'extension du pivot (3) étant supérieures aux dimensions de la piste de guidage (37) au niveau de l'extrémité de la piste de guidage (43), de façon à ce que la deuxième portion (11) limite un mouvement du pivot (3) par rapport à la deuxième partie de fermeture (21) perpendiculairement au plan de fermeture,
la piste de guidage (37) s'étendant jusqu'au bord de la deuxième partie de fermeture (21), l'ouverture d'entrée (41) étant réalisée au bord de la deuxième partie de fermeture (21),
et l'ouverture d'entrée (41) étant prévue entre l'extrémité de raccordement (23) et l'extrémité de couplage (25) sur un premier côté de la ligne de liaison (35) entre l'extrémité de raccordement (23) et l'extrémité de couplage (25),
**caractérisé en ce que** la deuxième partie de fermeture (21) comprend un autre dispositif de liaison (47) pour une autre sangle du dispositif à sangles,
l'autre dispositif de liaison (47) étant prévu sur un deuxième côté de la ligne de liaison (35), opposé au premier côté.

2. Fermeture selon la revendication 1, l'extrémité de la piste de guidage (43) et le pivot (3) étant conçus de façon à ce que la première partie de fermeture (1) puisse pivoter par rapport à la deuxième partie de fermeture (21) lorsque le pivot (3) se trouve à l'extrémité de la piste de guidage (43).

3. Fermeture selon la revendication 1 ou 2, des moyens de verrouillage (45) étant prévus, afin de maintenir le pivot dans la piste de guidage (37), de préférence au niveau de l'extrémité de la piste de guidage (43).

4. Fermeture selon la revendication 3, une saillie d'encliquetage (45) étant prévue dans la piste de guidage (37), de façon à ce que la largeur de la piste de guidage (37) soit réduite au niveau de la saillie d'encliquetage (45),
la deuxième partie de fermeture (21) étant conçue de façon à ce que la première portion du pivot (3) doive être déplacé de l'ouverture d'entrée (41) vers l'extrémité de la piste de guidage (43) et inversement devant la saillie d'encliquetage (45) contre une force élastique opposée.

5. Fermeture selon l'une des revendications 1 à 4, moyennant quoi, à proximité de la piste de guidage (37), au niveau de l'extrémité de la piste de guidage (43), de préférence à proximité de l'ensemble de la piste de guidage (37), une portion d'appui (39) avec une épaisseur unique est prévue et
la première portion (7) du pivot (3) présentant, dans sa direction axiale, une longueur qui correspond à l'épaisseur de la portion d'appui (39).

6. Fermeture selon la revendication 1, la projection de l'ouverture d'entrée (41) sur la ligne de liaison (35) entre l'extrémité de raccordement (23) et l'extrémité de couplage (25) est plus éloignée de l'extrémité de couplage (25) que la projection de l'extrémité de la piste de guidage (43) sur la ligne de liaison (35).

7. Fermeture selon la revendication 6, la piste de guidage (37) s'étendant avec une direction de courbure unique de l'ouverture d'entrée (41) vers l'extrémité de la piste de guidage (43).

8. Fermeture selon la revendication 7, la piste de guidage (37) étant conçue, au niveau de l'extrémité de la piste de guidage (43), de façon à ce que le mouvement (44) du pivot (3) hors de la position au niveau de l'extrémité de la piste de guidage (43) s'étende parallèlement à la ligne de liaison (35) entre l'extrémité de liaison (23) et l'extrémité de couplage (25).

9. Fermeture selon l'une des revendications 1 à 8, la première partie de fermeture (1) étant conçue de façon à ce que le pivot (3) s'étend en s'éloignant du dispositif de liaison et comprenant une extrémité libre (15) et
l'extrémité libre comprenant une cavité (17) s'étendant dans la direction axiale du pivot (5).

10. Orthèse avec un dispositif à sangles et avec une fermeture selon l'une des revendications 1 à 9, les dispositifs de liaison étant reliés avec les sangles du dispositif à sangles.

11. Orthèse selon la revendication 10, avec un dispositif à sangles circulaires (51, 57, 59), qui est conçu pour passer de manière circulaire et horizontale autour d'un patient et qui comprend une première et une deuxième portions (57, 59), qui sont prévues pour être reliées de manière amovible au niveau d'un point nodal (75),
avec un dispositif à sangles d'épaules (67), qui comprend une première extrémité (69) reliée avec le dispositif à sangles circulaires (51, 57, 59) et une deuxième extrémité, et qui est conçu pour s'étendre de la partie postérieure vers la partie antérieure au-dessus d'une épaule du patient jusqu'au point nodal (75),
la première portion (57) du dispositif à sangles circulaires (51, 57, 59) étant reliée au dispositif de liaison de la première partie de fermeture (1),
la deuxième portion (59) du dispositif à sangles circulaires (51, 57, 59) étant reliée au dispositif de liaison (27, 29, 31) de la deuxième partie de fermeture (21) et
le dispositif à sangles d'épaules (67) étant relié à l'autre dispositif de liaison (47) de la deuxième partie de fermeture (21).
